# EUROPEAN PATENT SPECIFICATION

(11) **EP 3 290 512 B1**
(45) Date of publication and mention of the grant of the patent: **25.10.2023**
(21) Application number: 17177108.2
(22) Date of filing: 21.06.2017
(51) Int. Cl.: C12N 5/0775, C12N 5/073, G01N 33/569

(54) **METHOD OF DETERMINING PURITY OF HUMAN UMBILICAL-CORD DERIVED MESENCHYMAL STEM CELLS**
VERFAHREN ZUR BESTIMMUNG DER REINHEIT VON MESENCHYMALEN STAMMZELLEN AUS NABELSCHNUR
PROCÉDÉ DE DÉTERMINATION DE LA PURETÉ DE CELLULES SOUCHES MÉSENCHYMATEUSES ISOLÉS DE CORDON OMBILICAL

(30) Priority: 31.08.2016 US 201662381934 P
(43) Date of publication of application: 07.03.2018
(73) Proprietor: Meridigen Biotech Co., Ltd., Taipei City 114 (TW)
(72) Inventor: HSUAN, Chang-Yo, 114 Taipei City (TW); LIN, Willie, 114 Taipei City (TW); SU, Yu-Chin, 114 Taipei City (TW); LIU, Wei-Ting, 114 Taipei City (TW); LI, Meng-Wei, 114 Taipei City (TW); DU, Mao-Kuang, 114 Taipei City (TW)
(74) Representative: Viering, Jentschura & Partner mbB Patent- und Rechtsanwälte

(56) References cited:
- EP-A1- 2 014 294
- WO-A1-2016/086403
- HYE JIN JIN ET AL: "Downregulation of Melanoma Cell Adhesion Molecule (MCAM/CD146) Accelerates Cellular Senescence in Human Umbilical Cord Blood-Derived Mesenchymal Stem Cells : CD146: A Negative Regulator of hUCB-MSC Senescence", STEM CELLS TRANSLATIONAL MEDICINE : SCTM, vol. 5, no. 4, 3 March 2016 (2016-03-03), pages 427-439, XP055434226, Durham ISSN: 2157-6564, DOI: 10.5966/sctm.2015-0109
- WING PUI TSANG ET AL: "CD146+ Human Umbilical Cord Perivascular Cells Maintain Stemness under Hypoxia and as a Cell Source for Skeletal Regeneration", PLOS ONE, vol. 8, no. 10, 18 October 2013 (2013-10-18), page e76153, XP055434265, DOI: 10.1371/journal.pone.0076153
- CHRISTINE ULRICH ET AL: "Human Placenta-Derived CD146-Positive Mesenchymal Stromal Cells Display a Distinct Osteogenic Differentiation Potential", STEM CELLS AND DEVELOPMENT, vol. 24, no. 13, 1 July 2015 (2015-07-01), pages 1558-1569, XP055434231, NL ISSN: 1547-3287, DOI: 10.1089/scd.2014.0465
- CRISAN M ET AL: "A Perivascular Origin for Mesenchymal Stem Cells in Multiple Human Organs", CELL STEM CELL, ELSEVIER, CELL PRESS, AMSTERDAM, NL, vol. 3, no. 3, 11 September 2008 (2008-09-11), pages 301-313, XP002611378, ISSN: 1934-5909, DOI: 10.1016/J.STEM.2008.07.003 [retrieved on 2008-09-10]
- COVAS D T ET AL: "Multipotent mesenchymal stromal cells obtained from diverse human tissues share functional properties and gene-expression profile with CD146<+> perivascular cells and fibroblasts", EXPERIMENTAL HEMATOLOGY, ELSEVIER INC, US, vol. 36, no. 5, 1 May 2008 (2008-05-01), pages 642-654, XP022619850, ISSN: 0301-472X, DOI: 10.1016/J.EXPHEM.2007.12.015 [retrieved on 2008-03-04]
- BENEDETTO SACCHETTI ET AL: "Self-Renewing Osteoprogenitors in Bone Marrow Sinusoids Can Organize a Hematopoietic Microenvironment", CELL, vol. 131, no. 2, 1 October 2007 (2007-10-01), pages 324-336, XP055434206, US ISSN: 0092-8674, DOI: 10.1016/j.cell.2007.08.025
- SORRENTINO A ET AL: "Isolation and characterization of CD146^+ multipotent mesenchymal stromal cells", EXPERIMENTAL HEMATOLOGY, ELSEVIER INC, US, vol. 36, no. 8, 1 August 2008 (2008-08-01) , pages 1035-1046, XP022940507, ISSN: 0301-472X, DOI: 10.1016/J.EXPHEM.2008.03.004 [retrieved on 2008-05-27]
- SHEN SHIH-PEI ET AL: "EphA2 is a biomarker of hMSCs derived from human placenta and umbilical cord", TAIWANESE JOURNAL OF OBSTETRICS AND GYNECOLOGY, vol. 54, no. 6, 15 December 2015 (2015-12-15), pages 749-756, XP029351081, ISSN: 1028-4559, DOI: 10.1016/J.TJOG.2015.10.012
- FENG-JUAN LV ET AL: "Concise Review: The Surface Markers and Identity of Human Mesenchymal Stem Cells", STEM CELLS, vol. 32, no. 6, 23 June 2014 (2014-06-23), pages 1408-1419, XP055152721, ISSN: 1066-5099, DOI: 10.1002/stem.1681
- CAPLAN A I: "All MSCs are pericytes?", CELL STEM CELL, ELSEVIER, CELL PRESS, AMSTERDAM, NL, vol. 3, no. 3, 11 September 2008 (2008-09-11), pages 229-230, XP002611380, ISSN: 1934-5909, DOI: 10.1016/J.STEM.2008.08.008 [retrieved on 2008-09-10]
- DOLORES BAKSH ET AL: "Comparison of Proliferative and Multilineage Differentiation Potential of Human Mesenchymal Stem Cells Derived from Umbilical Cord and Bone Marrow", STEM CELLS, vol. 25, no. 6, 1 June 2007 (2007-06-01), pages 1384-1392, XP055062689, ISSN: 1066-5099, DOI: 10.1634/stemcells.2006-0709

## Description

### FIELD OF THE INVENTION

The present invention pertains to a method of assessing the purity of human umbilical-cord derived mesenchymal stem cells (hUC-MSCs) in a cell culture.

### BACKGROUND OF THE INVENTION

Mesenchymal stem or stromal cells (MSCs) are multipotent cells of embryonic mesodermal origin, with a fibroblast-like morphology. These cells can differentiate into adipocytes, osteocytes, chondrocytes, neural lineage cells, and myocytes among other cell types depending on the stimuli and culture conditions. Although the plasticity of hMSCs and their role in tissue repair and regeneration have been extensively studied, it is their immunological trophic property that has gained the most interest recently [1-2]. Human mesenchymal stem cells have been isolated from a variety of tissues. The most frequently used source of MSCs is the bone marrow (BM). However, the isolation procedure is extremely invasive. To avoid the invasive isolation procedures, tissues such as human umbilical cord and placenta have been considered as good candidates since they are normally discarded after labor. The isolation of hMSCs from umbilical cord or placenta has proven to be efficient by previous studies [3].

MSCs are a subpopulation of a more complex cell composition of stromal cells contained in mesenchymal tissue. Due to the heterogeneous nature and the absence of known biomarkers specific for mesenchymal stem cells, it is a challenging task to define MSC phenotypes and characteristics [4-6]. The molecular components responsible for MSCs functionalities, in particular, those on the plasma membrane, remain largely unknown. In addition, lack of specific cell surface markers renders the cell culture at potential contamination risk with other cell types, in particular, those mature stromal cells such as fibroblasts, which, conversely, are abundant in mesenchymal tissue [4-6]. In the process of isolation of MSCs from placenta-derived tissues, non-MSCs, including fibroblasts, placenta-derived epithelial cells, and placenta-derived reticular cells, often coexist with MSCs during the in vitro cultivation. In particular, fibroblast is the main source of contamination.

Fibroblasts are considered mature mesenchymal cells that are particularly abundant in the connective tissue. Therefore, these cells are the most frequent contaminating cell phenotype present in many cell culture systems. Not only is it difficult to apply techniques which successfully eliminate fibroblasts from a culture, it is also particularly complex to distinguish MSCs from fibroblasts in the same culture. Fibroblasts and MSCs have an extremely similar morphological appearance; they both proliferate well and share many identical cell surface markers [7-8]. MSC are currently defined as plastic adherent, multipotent fibroblast-like cells expressing CD73, CD90, CD105 and negative for the hematopoietic markers CD14, CD34 and CD45 by the International Society of Cellular Therapy (ISCT). However, these properties and markers are also shared by fibroblasts. The current definition suggested by ISCT is thus incapable of distinguishing MSC from generic fibroblasts. Until now, the best way to distinguish MSCs from fibroblasts is based on the analysis of the functional properties of these two kinds of cells; MSCs retain multipotent stemness and immunomodulation capacity, while fibroblasts seem more limited in both of these functional properties.

Since Friedenstein's pioneering work in identification of MSCs [48], there are no distinct differences in culture-derivation methodology, morphology, and gene expression signature that consistently and unequivocally distinguish ex vivo culture-expanded MSC from fibroblasts [9-12]. Presently, there is no accepted criterion or single cell-surface marker for separating the MSCs from fibroblasts. Due to the fact that fibroblast is the common contaminant cell population in MSC culture when derived from placenta, a novel surface protein as a biomarker to distinguish MSCs from fibroblasts is crucial to ensure the homogeneity of primary culture of placenta-derived MSCs.

CD146 (melanoma cell adhesion molecule, MCAM), a surface marker of vascular endothelial cells, functions by facilitating cell-cell interactions and angiogenesis. Recent research reveals that some MSCs express CD146 [13-14, 16], and the expression levels of CD146 are associated with their abilities of self-renewal, or abilities of differentiation into adipocytes and osteogenesis [13, 15]. Further, MSCs isolated from different tissues show different CD146 expression levels: about 40% of MSCs isolated from bone marrow express CD146, and its expression levels decrease as the passage number increases [13-15], while only less than 10% of MSCs isolated from adipose tissues express CD146 [14].

US Patent No. 9,470,685 discloses a method of distinguishing MSCs from fibroblasts using a marker EphA2 expressed on the MSCs.

### BRIEF SUMMARY OF THE INVENTION

It was unexpectedly found in the present invention that human umbilical-cord derived mesenchymal stem cells (hUC-MSCs) can be distinguished in a cell culture, based on their expression levels of a specific surface marker, CD146 (melanoma cell adhesion molecule, MCAM).

In certain embodiments of the present invention, the cells derived from a human umbilical-cord derived tissue are cultured in a culture medium for MSC.

In one aspect, the present invention provides a method of assessing purity of human umbilical-cord derived mesenchymal stem cells (hUC-MSCs) in a cell culture. Said method comprises the step of determining the percentage of cells expressing CD146 in the culture.

According to the present invention, the purity of MSCs has a positive correlation with the percentage of cells expressing CD146. In preferred embodiments of the present invention, the percentage of cells expressing CD146 is determined by a flow cytometry. According to the invention, the purity of hUC-MSCs is calculated by the following equation: Y = SX + I, where Y is the purity (%) of MSCs, X is the percentage of cells expressing CD146, S is 1.206, and I is -23.32.

It is to be understood that both the foregoing general description and the following detailed description are exemplary and explanatory only and are not restrictive of the invention.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments which are presently preferred.

In the drawings:
**Fig. 1** shows the linear regression between the purity (%) of MSCs and the percentage of cells expressing CD146 in a cell culture.
**Fig. 2A** shows the CD146 expression level of CD146^{high} MSCs and CD146^{low} MSCs. **Fig. 2B** shows the results of adipogenesis differentiation from CD146^{high} MSCs and CD146^{low} MSCs.
**Fig. 3** shows the results of PBMC proliferation assay.
**Fig. 4** shows the levels of hepatocyte growth factor (HGF) in conditioned medium of CD146^{high} MSCs and CD146^{low} MSCs.
**Fig. 5** shows the relative levels of cellular ROS, mitochondrial ROS and β-galactosidase (a senescence marker) in CD146^{high} MSCs and CD146^{low} MSCs.

### DETAILED DESCRIPTION OF THE INVENTION

The present invention is based on the unexpected finding that through a cell sorting by surface marker CD146, human umbilical-cord derived mesenchymal stem cells (hUC-MSCs) can be distinguished in a cell culture.

In certain embodiments of the present invention, the method further comprises the preliminary steps of: collecting the hUC-MSCs and the fibroblasts from the human umbilical-cord tissue; and culturing the MSCs and the fibroblasts in a culture medium to prepare the cell culture.

According to the present invention, the cells are freshly derived, obtained or collected from a human umbilical-cord tissue following a protocol known in the art, for example, that of Shen *et al.* [21]. In certain preferred embodiments, the cells derived from a human umbilical-cord tissue are then cultured in a culture medium for hUC-MSCs. A standard medium for MSC comprises α minimal essential medium (with different versions of modification), fetal bovine serum (FBS), L-glutamine, and basic fibroblast growth factor (bFGF) [3, 16-20].

In carrying out the methods of the present invention, a cell culture derived from a human umbilical-cord tissue is subjected to a cell sorting by CD146. The cell sorting may be performed through a technique known or to be developed in the art, for example, an antibody-based or a nucleotide-based isolation method. Preferably, the cell sorting is performed by an antibody-based magnetic cell sorting. For example, the MACS method (MACS^{®} Technology, Miltenyi Biotec). In addition, the cell sorting may be performed through a flow cytometry method, e.g. an antibody-based or a nucleotide-based flow cytometry.

Further, it was unexpected found in the present invention that the purity of hUC-MSCs has a positive correlation with the percentage of cells expressing CD146.

Accordingly, the present invention in a further aspect provides a method of assessing purity of human umbilical-cord derived mesenchymal stem cells (hUC-MSCs) in a cell culture. Said method comprises the step of determining the percentage of cells expressing CD146 in the culture.

In preferred embodiments of the present invention, the percentage of cells expressing CD146 is determined by a flow cytometry.

In one embodiment, the purity of hUC-MSCs is calculated by the following equation: Y = SX + I, where Y is the purity (%) of hUC-MSCs, X is the percentage of cells expressing CD146, S is 1.206, and I is -23.32.

According to certain embodiments of the present invention, the population of hUC-MSCs isolated by a method of the present invention has a higher differentiation ability. In one embodiment, the population of MSCs has a higher adipogenesis ability.

According to certain embodiments of the present invention, the population of MSCs isolated by a method of the present invention elicits higher anti-inflammatory effects. In one embodiment, the population of MSCs exhibits a higher ability in inhibiting PBMC proliferation.

According to certain embodiments of the present invention, the population of MSCs isolated by a method of the present invention has a lower degree of senescence. In some embodiments, the population of MSCs expresses a lower level of cellular ROS, mitochondrial ROS, or beta-galactosidase.

According to certain embodiments of the present invention, the population of MSCs isolated by a method of the present invention secretes a higher amount of growth factors. In one embodiment, the population of MSCs secretes a higher amount of a hepatocyte growth factor (HGF).

It is to be understood that both the foregoing general description and the following detailed description are exemplary and explanatory only and are not restrictive of the invention.

The present invention is further illustrated by the following examples, which are provided for the purpose of demonstration rather than limitation.

### Examples

### Example 1: Assessment of purity of MSCs

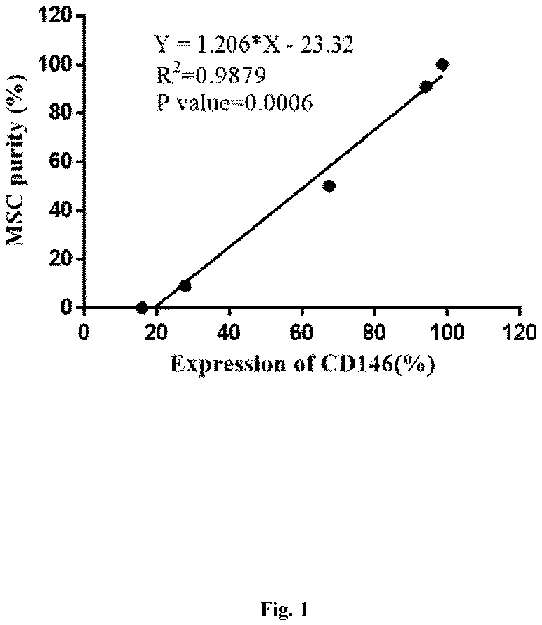

MSCs derived from umbilical cord were mixed with fibroblasts in Eppendorf tubes by following ratios (MSC: fibroblasts in cell number): 2×10⁵ : 0, 1.8×10⁵ : 2×10⁴, 1×10⁵ : 1×10⁵, 2×10⁴ : 1.8×10⁵, or 0 : 2×10⁵. CD146+ population in each Eppendorf tube was then analyzed by flow cytometry. Subsequently, the relationship between the purity (%) of MSCs and the percentage of cells expressing CD146 was analyzed by simple linear regression, results of which are shown in **Fig. 1**. The regression equation is Y = 1.206X - 23.32, where Y is the purity (%) of MSCs and X is the percentage of cells expressing CD146. The results demonstrated that there is a linear relationship between the expression level of CD146 and the purity of MSCs.

### Example 2: CD146^{high} MSCs exhibits higher differentiation ability than CD146^{low} MSCs

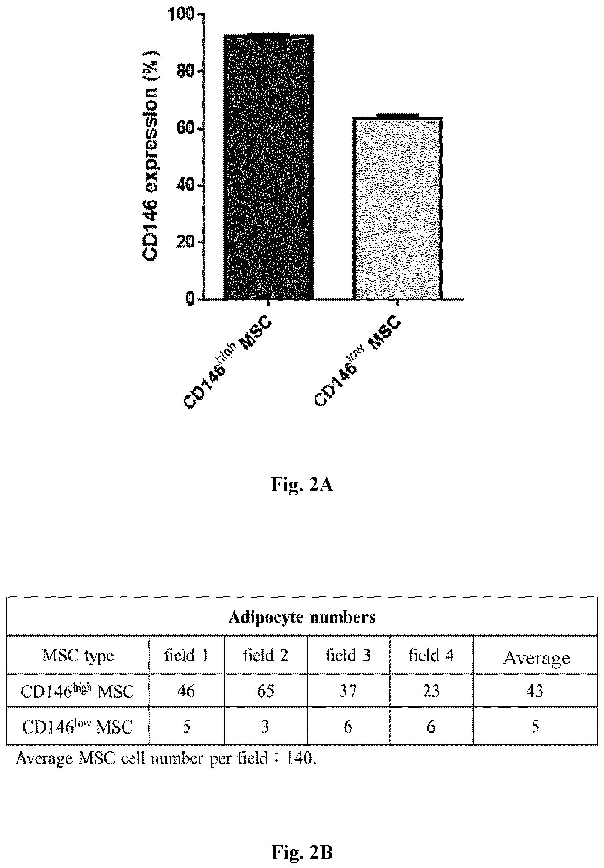

MSCs derived from different umbilical cords were examined for expression of CD146 using flow cytometry. Two groups of MSCs were obtained: CD146^{high} MSCs and CD146^{low} MSCs **(****Fig. 2A****).** 2 × 10⁵/well MSCs were seeded into 12-well plate, cultured overnight, and then cultured in adipocyte differentiation basal medium (InvitroGen) supplemented with adipogenesis supplement (InvitroGen). After culture for 14 days, the two groups of MSCs were fixed formalin, stained with Oil Red, and observed under a microscope to count the number of adipocytes. As shown in **Fig. 2B**, CD146^{high} MSCs exhibited higher adipogenesis ability.

### Example 3: CD146^{high} MSCs elicits higher anti-inflammatory effects than CD146^{low} MSCs

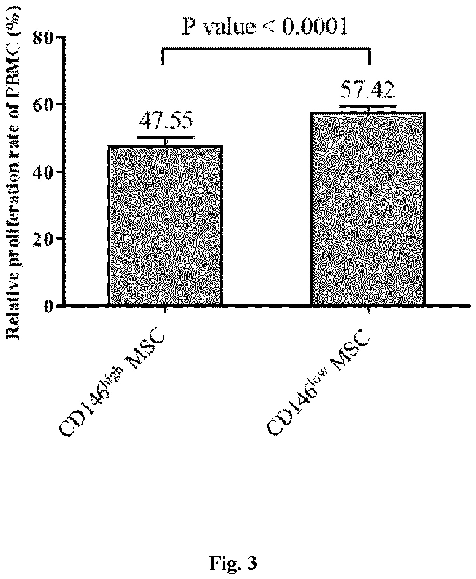

CD146^{high} MSCs and CD146^{low} MSCs were obtained as described in Example 2. The immune modulation capability of MSC was analyzed by PBMC proliferation assay. Carboxyfluorescein succinimidyl ester (CFSE) labeled PBMC is stimulated by phytohemagglutinin (PHA) and co-cultured with MSCs. The percentage of proliferated PBMCs was analyzed by FACS. The proliferation rate of PBMC was determined by assessing the reduction of the intensity of the fluorescent cell permeable dye CFSE. The results show that CD146^{high} MSCs were more effective in inhibiting PBMC proliferation than CD146^{low} MSCs **(****Fig. 3****),** suggesting that CD146^{high} MSCs elicit higher anti-inflammatory effects.

### Example 4: CD146^{high} MSCs secretes a higher amount of growth factors than CD146^{low} MSCs

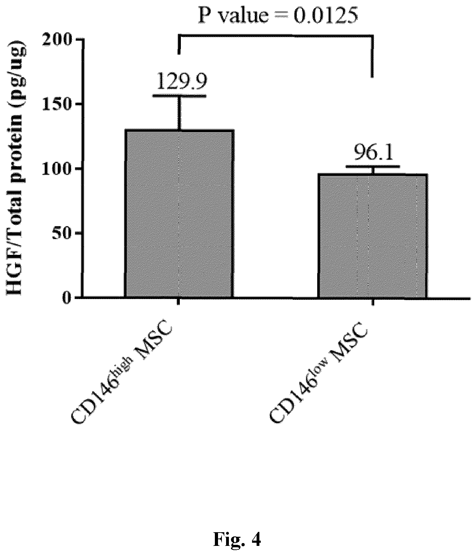

CD146^{high} MSCs and CD146^{low} MSCs were obtained as described in Example 2. Hepatocyte growth factor (HGF) level in conditioned medium from both groups was analyzed by ELISA and MSC total protein was analyzed by BCA assay. HGF level is expressed as a ratio of HGF amount to MSC total protein amount. As shown in **Fig. 4**, CD146^{high} MSCs secreted a significantly higher amount of HGF than CD146^{low} MSCs.

### Example 5: CD146^{high} MSCs have a lower degree of senescence than CD146^{low} MSCs

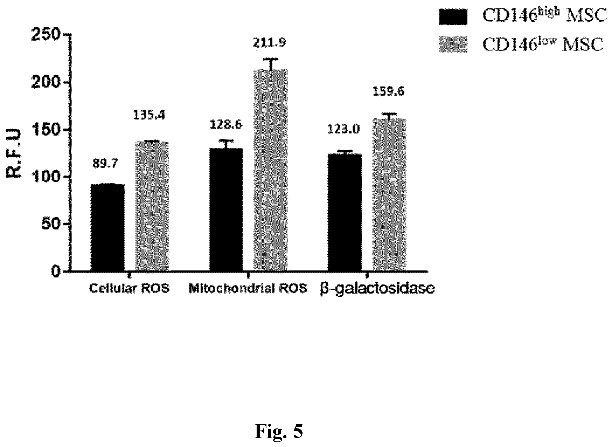

CD146^{high} MSCs and CD146^{low} MSCs were obtained as described in Example 2. Higher levels of cellular ROS, mitochondrial ROS and beta-galactosidase are considered to be related to senescence. In the present experiment, cellular ROS level and mitochondrial ROS level were determined using commercial kits (DCFDA of Sigma, and MitoSox of Invitrogen, respectively). The level of senescence-associated beta-galactosidase was determined using C12FDG (Molecular Probes). The results show that the levels of cellular ROS, mitochondrial ROS and beta-galactosidase in CD146^{high} MSCs were lower than in CD146^{low} MSCs **(****Fig. 5****),** suggesting that CD146^{high} MSCs have a lower degree of senescence than CD146^{low} MSCs.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the appended claims.

### References:

[1] Stagg Jet al., Curr Mol Med. 13(5):856-67 (2013).
[2] Casado JG et al., Stem Cell Rev. 9(2):184-9 (2013).
[3] Fukuchi Y et al., Stem Cells. 2004;22(5):649-58.
[4] Javazon EH et al., Exp Hematol. 32(5):414-25 (2004).
[5] Nombela-Arrieta C et al., Nat Rev Mol Cell Biol. 12(2):126-31 (2011).
[6] Keating A., Cell Stem Cell. 10(6):709-16 (2012).
[7] Linge C et al., Exp Cell Res 1989, 185:519-528.
[8] Lorenz K et al., Exp Dermatol 2008, 17:925-932.
[9] Blasi et al., Vascular Cell 2011, 3:5.
[10] Covasa *et al.,* Volume 36, Issue 5, Pages 642-654 (2008).
[11] Muzlifah A. Haniffa et al., Haematologica 94:258-263 (2009).
[12] Pittenger et al., Science Vol. 284 no. 5411 pp. 143-147(1999).
[13] Lv F.J. et al., Stem Cells. 2014;32(6):1408-19.
[14] Dmitrieva R.I.et al., Cell Cycle. 2012;11(2):377-83.
[15] Halfon S. et al., Stem Cells Dev. 2011;20(1): 53-66.
[16] Miao Z et al., Cell Biol Int. 2006 Sep;30(9):681-7.
[17] Malek A et al., J Stem Cells. 2011;6(2):75-92.
[18] Cavallo C et al., J Cell Biochem. 2011 May;112(5):1418-30. doi: 10.1002/jcb.23058.
[19] Shalini Vellasamy et al., World J Stem Cells. Jun 26, 2012; 4(6): 53-61.
[20] Luan X et al., Tissue Cell. 2013 Feb;45(1):32-8. doi: 10.1016/j.tice.2012.09.002. Epub 2012 Oct 27.
[21] Shen et al., Taiwan J Obstet Gynecol. 2015 Dec;54(6):749-56. doi: 10.1016/j.tjog.2015.10.012.

## Claims

1. A method of assessing the purity of human umbilical-cord derived mesenchymal stem cells (hUC-MSCs) in a cell culture, comprising determining the percentage of cells expressing CD146 in the culture;
wherein the purity of hUC-MSCs is calculated by the equation shown below:
Y = SX + I, where Y is the purity (%) of hUC-MSCs, X is the percentage of cells expressing CD146, S is 1.206, and I is -23.32.

2. The method of claim 1, wherein the percentage of cells expressing CD146 is determined by flow cytometry.

3. The method of claim 1, wherein the purity of hUC-MSCs has a positive correlation with the percentage of cells expressing CD146.

## Patentansprüche

1. Verfahren zur Bewertung der Reinheit von aus menschlicher Nabelschnur stammenden mesenchymalen Stammzellen (hUC-MSZs) in einer Zellkultur, das die Ermittlung des Prozentsatzes der CD146 exprimierenden Zellen in der Kultur umfasst;
wobei die Reinheit der hUC-MSZs durch die unten dargestellte Gleichung berechnet wird:
Y = SX + I, wobei Y die Reinheit (%) der hUC-MSZs ist, X ist der Prozentsatz der Zellen, die CD146 exprimieren, S ist 1,206 und I ist -23,32.

2. Verfahren gemäß Anspruch 1, wobei der Prozentsatz der Zellen, die CD146 exprimieren, durch Durchflusszytometrie ermittelt wird.

3. Das Verfahren gemäß Anspruch 1, wobei die Reinheit der hUC-MSZs eine positive Korrelation mit dem Prozentsatz der CD146 exprimierenden Zellen aufweist.

## Revendications

1. Procédé d'évaluation de la pureté de cellules souches mésenchymateuses dérivées de cordon ombilical humain (hUC-MSC) dans une culture cellulaire, comprenant une détermination du pourcentage de cellules exprimant CD146 dans la culture ;
dans lequel la pureté des hUC-MSC est calculée par l'équation indiquée ci-dessous :
Y = SX + I, dans laquelle Y est la pureté (%) des hUC-MSC, X est le pourcentage de cellules exprimant CD146, S est 1,206 et 1 est -23,32.

2. Procédé selon la revendication 1, dans lequel le pourcentage de cellules exprimant CD146 est déterminé par cytométrie en flux.

3. Procédé selon la revendication 1, dans lequel la pureté des hUC-MSC présente une corrélation positive avec le pourcentage de cellules exprimant CD146.
